# EUROPEAN PATENT APPLICATION

(11) **EP 2 870 975 A1**
(43) Date of publication of application: **13.05.2015**
(21) Application number: 13191842.7
(22) Date of filing: 06.11.2013
(51) Int. Cl.: A61M 5/32

(54) **Sheath comprising a lock ring and safety syringe comprising said sheath**

(71) Applicant: SOFIC (Sté Française d'Instruments de Chirurgie), 81200 Mazamet (FR)
(72) Inventor: The designation of the inventor has not yet been filed
(74) Representative: Icosa

(57) **Abstract**

The present invention relates to a protective sheath (1) for a cannula (4) arranged on a hub which can be fitted onto a syringe characterized in that:
- said sheath (1) comprises a lock ring (2) located on the outer surface of said sheath (1);
- said lock ring (2) is rotatable with respect to the sheath (1) between three positions:
- a position before use (PBU);
- a position during use (PU); and
- a position after use (PAU).

The present invention also relates to an injection system comprising a sheath according to the present invention; and to a syringe comprising a sheath according to the present invention or an injection system according to the present invention.

## Description

### FIELD OF INVENTION

The present invention relates to a sheath, comprising a rotating lock ring, protecting a cannula; the sheath is primarily designed to prevent or minimize accidental needlestick injuries. The present invention also relates to an injection system comprising said sheath; especially the present invention relates to a safety syringe comprising said sheath, and more particularly to a safety syringe adapted to receive a medication-containing cartridge.

### BACKGROUND OF INVENTION

Needlestick injuries frequently occur among healthcare workers, introducing high risk of blood-borne pathogen infections for surgeons, assistants, and nurses, such as HIV, hepatitis B, hepatitis C or viral hemorrhagic fevers. In a UK report, 37% of nurses reported that they have sustained a needle-stick injury at some stage during their career (Prevention CfDCa, Overview: Risks and Prevention of Sharps Injuries in Healthcare Personnel, CDC, Atlanta, Ga, USA, 2004). These results have further to be read taking into account that not all needle-stick injuries are reported, and that the rate of detection may be low. In a study investigating the use of blunt needles during obstetrical laceration repair surgeries (Wilson et al., "The use of blunt needles does not reduce glove perforations during obstetrical laceration repair", Am. J. Obstet. Gynecol., 2008, 199(6):641.e1-3), only 11% of glove perforations were detected by the physician in a study.

Strategies are available in response to these issues, including education of healthcare workers on the risks and precautions, reduction of invasive procedures, management of exposures and use of safer devices.

Among the safest devices, an efficient way to protect a healthcare worker from being pricked by a needle is the use of safety syringes. Such syringes have the particularity to comprise a sheath protecting the needle, which can be retracted or extended with respect to the sheath. In a retracted position, the needle is completely covered and secured by the sheath. In an extended position, the needle projects outside the sheath.

A major issue when designing such safety syringes, further to securing the needle, is the ease of use of said safety syringes. Even though the protection sheath may enhance safety, its volume may make the syringe uneasy and cumbersome to manipulate.

Several safety syringes comprising a sheath have been proposed in the literature. However, none of them describes a sheath according to the invention, designed for an improved convenience and an improved safety for the healthcare worker.

For example, International patent application WO 2006/105006 discloses a safety syringe including locking positions, formed by an inner sleeve receiving a cartridge filled with liquid, an outer sleeve through which said inner sleeve is telescopically reciprocated, and a plunger assembly that is attached to the inner sleeve and used to eject the liquid. This device is capable of both intermittent locking during use as well as permanent locking after use (page 5, lines 2-3), performed by pushing inward a tab which engages permanently a hole. However, there is no specific retracted position before use RBU.

US patent application 2011/0082428 relates to a safety structure for covering a syringe needle including a safety sleeve and a hub. The safety sleeve is fitted around the hub and provided with an axial sliding slot, a locating slot laterally extended from the sliding slot and a locating hole located at a bottom of the locating slot. The protective sheath comprises a "retracted position before use" in the locating slot, an "ejection position" in the sliding slot and a "retracted position after use" in the locating hole. However, this device does not provide a "retracted position during use". Moreover, this safety structure does not provide a one-way path for the male means of the hub through the female means of the safety sleeve: backtracks are indeed needed in order to access to the various retracted positions. Thus said safety structure lacks of convenience and safety.

US patent US 4,994,045 discloses a safety syringe including a conventional syringe, a locating ring and an elongated and tubular sheath. This device is capable of both intermittent and permanent positions where the needle is secured, allowing the user to extend or retract the tubular sheath at will during use, and to definitely secure the needle after use. However, the various retracted positions are not transversally aligned (see figure 2 of US 4,994,045), resulting in a non-optimized sheath length as described further in the description of the invention.

European Patent EP 1 603 612 discloses a preservative sheath for an injection needle or cannula arranged on a cannula-holding support which can be fitted onto a syringe. The sheath comprises guide means, which may be ramps, allowing the cannula to be in a retracted position before use, a retracted position during use, a retracted position after use and an ejection position. However, the various retracted positions are not transversally aligned, resulting in a non-optimized sheath length as described further in the description of the invention.

Therefore, there is still a need in the art for a needle protective sheath improving:
- the healthcare worker's convenience by providing a sheath with optimized length and volume; and
- the safety by providing a user friendly sheath.

In order to overcome the disadvantages of the prior art, the needle protective sheath of the present invention comprises a lock ring cooperating with the protective sheath between three positions: a position before use, a position during use, and a position after use. In each of said positions the hub is located at the same place. This feature improves the syringe both from the point of view of the functional utility and the cost of manufacture.

Moreover in order to improve the safety of the device (i.e. the sheath comprising a rotating lock ring), said three positions are achieved thanks to a rotating lock ring which is instinctively rotated by the user. Indeed in the device of the present invention an easy rotation is required for reaching said three positions. In the devices of the prior art the user had to move the syringe, and especially a protrusion of the hub united with the syringe, along a female means pathway through the different positions of the sheath; thus translations and rotations were needed. The handling of the present invention is therefore easier and safer.

### SUMMARY

This invention relates to a protected sheath for a cannula arranged on a hub which can be fitted onto a syringe characterized in that:
- said sheath comprises a lock ring located on the outer surface of said sheath;
- said lock ring is rotatable with respect to the sheath between three positions:
- a position before use (PBU);
- a position during use (PU); and
- a position after use (PAU).

According to a first embodiment, the sheath further comprises:
- an ejection means (EM) intended to cooperate with a protrusion of the hub so that whole or part of the cannula could protrude out of the sheath;
- an opening, in line with the ejection means (EM), for receiving the protrusion of the hub; and
   wherein:
   - in the position before use (PBU), the lock ring temporarily prevents access between the opening and the ejection means (EM);
   - in the position during use (PU), the lock ring enables access between the opening and the ejection means (EM); and
   - in the position after use (PAU), the lock ring definitely prevents access between the opening and the ejection means (EM).

According to an embodiment, the whole length of the cannula is inside said sheath when the protrusion of the hub is located in the opening of the sheath.

According to another embodiment:
- the lock ring further comprises a first, a second and a third recess, located on the inner surface of the lock ring, and
- the sheath further comprises a protrusion on the outer surface for cooperating with said recesses;
   and wherein,
- in the position before sue (PBU) the protrusion on the outer surface of the sheath is located in a first recess on the inner surface of the lock ring;
- in the position during use (PU) the protrusion on the outer surface of the sheath is located in a second recess on the inner surface of the lock ring; and
- in the position after use (PAU) the protrusion on the outer surface of the sheath is located in a third recess on the inner surface of the lock ring.

According to another embodiment, the sheath protrusion has a ramp-shaped cross-section, the wall between the first recess and the third recess has a ramp-shaped cross section, and the wall between the first recess and the second recess has a curved cross-section.

According to another embodiment, the wall between the first recess and the second recess prevents unintentional displacement of the sheath protrusion between said recesses, but enable intentional displacement by application of a torque by the user; and the wall between the first recess and the third recess prevents unintentional and intentional displacement of the sheath protrusion between said recesses, once the sheath protrusion is in the third recess.

According to an embodiment, the sheath further comprises a sliding opening, located transversally between the opening and the ejection means (EM), for receiving a protrusion located on the inner surface of the lock ring; and wherein:
- when the lock ring is in the position before use (PBU) the protrusion on the inner surface of the lock ring, located in the sliding opening, prevents access between the opening and the ejection means (EM);
- when the lock ring is rotated in one direction and reaches the position during use (PU) the protrusion on the inner surface of the lock ring, located in the sliding opening, enables access between the opening and the ejection means (EM); and
- when the lock ring is rotate in the opposite direction, the lock ring reaches the position before use (PBU), and by further rotation, the position after use (PAU) and the protrusion on the inner surface of the lock ring, located in the sliding opening, prevents access between the opening and the ejection means (EM).

According to an embodiment, the sheath further comprises anti-slipping means, including at least one rib for preventing dismounting of the hub from said sheath.

According to an embodiment, the distal end of said sheath is conical or beveled.

According to an embodiment, the sheath further comprises gripping means which are ribs covered by a grinding or an erosion grain such as VDI 20 to 30, located on the external surface of said sheath.

The present invention also relates to an injection system comprising a sheath according to the present invention, a hub comprising at least one protrusion adapted to cooperate with the sheath, and, optionally, a cannula protruding at both ends of the hub.

The present invention evenly relates to a syringe comprising a sheath according to the present invention or an injection system according to the present invention.

According to an embodiment, the syringe is designed to hold a cartridge which is immobilized in axial direction at the neck of said cartridge, via a blocking means.

According to an embodiment, said syringe is a disposable syringe having a precut located substantially at the distal end of the syringe, made of a series of perforations or one or more circumferential grooves.

### DEFINITIONS

In the present invention, the following terms have the following meanings:
- **"About"** preceding a value means plus or less 10% said value.
- An **"anti-return"** means is here a means allowing the passage of a male means within a female means from a first position to a second position, and preventing the passage from the second position to the first position.
- **"Cannula"** refers to a tube that can be inserted into the body, generally for the delivery or removal of a fluid.
- **"Cartridge"** refers generally to a small sealed vial, used to contain and/or preserve a pharmaceutical composition.
- The hub is **"definitely locked"** within the sheath if said hub may move with regard to said sheath only if a brutal external force is applied, for example a force able to break the device.
- **"Distal end"** of a part refers to the farthest end of this part, relative to a point of origin. Most often the point of origin is the user and the distal end is the closest end to the tissue area in which the medicine will be injected.
- **"Distinct"** means physically separate. **"Distinct positions"** refer thus to physically or materially separate position.
- **"Ejection position"** refers here to a position of the hub when the cannula protrudes the sheath at least partially, rendering possible to insert the cannula in a patient for an injection for example.
- **"Female means"** refers to any cavity or hole in any shape, such as a groove, a slot, a slit, or a notch, at the surface or through a material, cooperating with a male means. In other words, male means may be inserted into female. In the present invention slots, recesses, grooves and openings are considered as female means. The wording differs in order to facilitate the understanding the present invention.
- **"Hub"** refers to a piece capable of connecting a cannula to the distal end of a syringe according to the invention, or more generally to the distal end of any syringe, wherein the distal end of a syringe is the end farthest to the piston.
- The verb **"guiding"** used with male and female means, means that said female means provide a path via hole or grooves for instance, in which said male means may move freely, and prevent said male means to step out of said path, via walls or inclined planes for instance.
- **"Insertion"** of the protrusion of the hub refers here to the insertion of said protrusion within the female means of the sheath, said female means being possibly designed as to facilitate the insertion of said protrusion. For example, female means designed as insertion means may be provided with a special opening in which male means are easily inserted. Insertion means may be also provided with an anti-return means, so that once inserted, male means cannot be removed from the female means.
- **"Integrally formed"** refers to a part in one piece, or to a part comprising several components, wherein any components making up the part have been rendered securely jointed.
- A **"locking means"** refers here to a part which prevents male means to step out of a defined position.
- **"Male means"** refers to any part constituted by matter, cooperating with female means. In other words male means may be inserted into female. In the present invention protrusions, projections, keys are considered as male means. The wording differs in order to facilitate the understanding the present invention.
- **"Pharmaceutical composition"** refers to a composition comprising an active principle in association with a pharmaceutically acceptable vehicle or excipient. A pharmaceutical composition is for therapeutic use, and relates to health. Especially, a pharmaceutical composition may be indicated for treating or preventing a disease. According to the invention, the term **"treating a disease"** refers to reducing or alleviating at least one adverse effect or symptom of a disease, disorder or condition associated with a deficiency in an organ, tissue or cell function. The expression **"Preventing a disease"** or **"Inhibiting the development of a disease"** refers to preventing or avoiding the occurrence of symptom.
- **"Priming position"** refers to a position in which the user releases the air from the syringe and depresses the plunger until a small amount of the liquid medium comes out.
- **"Protective sheath"** or **"sheath"** refers here to an element preventing an easy access to another element able to hurt. It may be for example a tube preventing fingers to access a needle, which may be retracted or extended.
- **"Proximal end"** of a part refers to the closest end of this part, relative to a point of origin. Most often the point of origin is the user and the proximal end is the farthest end from the tissue area in which the medicine will be injected.
- **"Retracted position"** refers here to a position of the hub wherein the cannula is protected within the sheath, preventing fingers for example to access said cannula. In the present invention, in the retracted position, the protrusion of the hub is located in the opening of the sheath.
- **"Lock ring"** refers here to any hollow cylindrical means designed to rotate with respect to the sheath, such that for example a ring, a collar or any other means that one skilled in the art would find suitable.
- **"Rotational movement"** refers here to the rotation of the hub within the sheath, around the main axis of the sheath (i.e. the axis of the cylinder).
- **"Standard syringe"** refers to a syringe comprising a barrel, wherein an injectable composition may be directly loaded.
- **"Substantially"** at the end of a part means that the location ranges from 0.01 to 25%, preferably from 1 to 20% of the total length of said part from said end.
- **"Syringe"** refers to any device comprising a container or a means intended to receive a container, an opening and a piston, wherein said piston is used to push the content of said container through said opening.
- The hub is here **"temporary locked"** within the sheath if the movement of said hub with regard to said sheath cannot spontaneously occur under common natural forces such as gravity, but requires an external force.
- **"Translation movement"** refers here to a longitudinal movement, having the same direction than the axis of the cylinder.
- **"Transversally aligned"** refers here to the property of various points on a tube having a longitudinal axis X, wherein each point has the same coordinate along the X axis. **"Substantially transversally aligned"** refers here to the property of various points on a tube having a longitudinal axis X, wherein each point has the same coordinate plus or minus 3 mm, preferably plus or minus 2.5 mm, more preferably plus or minus 1.5 mm, even more preferably plus or minus 0.5 mm, still more preferably plus or minus 0.25 mm along the longitudinal axis X.

### BRIEF DESCRIPTION OF THE DRAWINGS

The following detailed description will be better understood when read in conjunction with the drawings. For the purpose of illustrating, the device is shown in the preferred embodiments. It should be understood, however that the application is not limited to the precise arrangements, structures, features, embodiments, and aspect shown. The drawings are not drawn to scale and are not intended to limit the scope of the claims to the embodiments depicted.
**Figure 1a** is a perspective view showing the sheath according to one embodiment of the invention in which the lock ring is in the position before use.
**Figure 1b** is a perspective view showing the sheath according to one embodiment of the invention in which the lock ring is in the position during use.
**Figure 1c** is a perspective view showing the sheath according to one embodiment of the invention in which the lock ring is in the position after use.
**Figure 2a** is a side view showing the sheath according to one embodiment of the invention in which the lock ring is in the position before use.
**Figure 2b** is a side view showing the sheath according to one embodiment of the invention in which the lock ring is in the position during use.
**Figure 2c** is a side view showing the sheath according to one embodiment of the invention in which the lock ring is in the position after use.
**Figure 3a** is a cross-sectional view along A-A showing the sheath according to one embodiment of the invention in which the lock ring is in the position before use.
**Figure 3b** is a cross-sectional view along A-A showing the sheath according to one embodiment of the invention in which the lock ring is in the position during use.
**Figure 3c** is a cross-sectional view along A-A showing the sheath according to one embodiment of the invention in which the lock ring is in the position after use.
**Figure 4a** is a cross-sectional view along B-B showing the sheath according to one embodiment of the invention in which the lock ring is in the position before use.
**Figure 4b** is a cross-sectional view along B-B showing the sheath according to one embodiment of the invention in which the lock ring is in the position during use.
**Figure 4c** is a cross-sectional view along B-B showing the sheath according to one embodiment of the invention in which the lock ring is in the position after use.
**Figure 5a** is a side view showing the sheath according to one embodiment of the invention in which the lock ring is in the position before use.
**Figure 5b** is a side view showing the sheath according to one embodiment of the invention in which the lock ring is in the position during use.
**Figure 5c** is a side view showing the sheath according to one embodiment of the invention in which the lock ring is in the position after use.
**Figure 6a** is a cross-sectional view along C-C showing the sheath according to one embodiment of the invention in which the lock ring is in the position before use.
**Figure 6b** is a cross-sectional view along C-C showing the sheath according to one embodiment of the invention in which the lock ring is in the position during use.
**Figure 6c** is a cross-sectional view along C-C showing the sheath according to one embodiment of the invention in which the lock ring is in the position after use.
**Figure 7** is an exploded view of the sheath according to one embodiment of the invention.
**Figure 8** is a cross-sectional view showing a cartridge held by a self demolding clip inside a syringe according to the invention.
**Figure 9** is a cross-sectional view showing a cartridge held by the back of a syringe according to the invention.
**Figure 10** is a cross-sectional view showing a cartridge being inserted into a syringe according to the invention.
**Figure 11** is a cross-sectional view showing a cartridge inserted into a syringe according to the invention.

### REFERENCES

- **1**: Sheath
- **1.1**: Sheath protrusion
- **1.2**: Opening for hub protrusion
- **1.4**: Sliding opening on the sheath for lock ring protrusion
- **1.5**: Longitudinal slot of the sheath
- **1.6**: Beveled portion
- **1.7**: Gripping portion
- **1.8**: Distal end
- **1.9**: Proximal end
- **1.10**: Internal surface
- **1.11**: External surface
- **2**: Lock ring
- **2.1**: Lock ring protrusion
- **2.2.1**: Lock ring temporary protrusion
- **2.2.2**: Lock ring final protrusion
- **2.3**: Opening, through the lock ring, adjacent to the lock ring protrusion
- **2.4.1**: First recess on the lock ring for sheath protrusion
- **2.4.2**: Second recess on the lock ring for sheath protrusion
- **2.4.3**: Third recess on the lock ring for sheath protrusion
- **3**: Hub
- **3.1**: Hub protrusion
- **3.2**: Distal end of hub
- **3.3**: Proximal end of the hub
- **3.4**: Reception means
- **4**: Cannula
- **4.1**: Distal end of the cannula
- **4.2**: Proximal end of the cannula
- **5**: Cartridge
- **5.1**: Neck of the cartridge
- **6.1**: Self demolding clip
- **6.2**: Back of the syringe
- **6.3**: Blocking means
- **IM**: Injection means
- **RP**: Retracted position
- **PBU**: Position before use
- **PU**: Position during use
- **PAU**: Position after use
- **EM**: Ejection means
- **EP**: Ejection position

### DETAILED DESCRIPTION

### Protective sheath

The present invention relates to a protective sheath **1** for a cannula **4** arranged on a hub **3** which can be fitted onto a syringe, allowing a safe use of said cannula **4** without the risk of being accidentally pricked.

### Lock ring

The sheath of the present invention comprises a lock ring **2.** In one embodiment, the lock ring **2** is mounted on the outer surface of the sheath **1.11.** In one embodiment, the internal diameter of said lock ring **2** is equal or slightly superior to the external diameter of the sheath **1.** In another embodiment, the lock ring **2** has a length preferably ranging from 1.5 to 30 mm, preferably from 3 to 25 mm, more preferably from 5 to 20 mm. In one embodiment, the thickness of the lock ring **2** is comprised between 0.2 and 15 mm, preferably between 0.5 and 10 mm, more preferably between 2 and 8 mm.

According to an embodiment, the lock ring **2** is not mounted on the inner surface of the sheath **1.10.** According to another embodiment, the lock ring **2** is mounted on the inner surface of the sheath **1.10.**

### Lock ring - positions

According to one embodiment, the sheath of the present invention **1** comprises lock ring **2** cooperating with the protective sheath **1** between three positions: a position before use **PBU,** a position during use **PU,** and a position after use **PAU.**

The various positions of the lock ring **2** contribute to the healthcare worker's safety, in that:
- the position before use **PBU** prevents needlestick injuries (NSIs) before use of cannula **4;**
- the position during use **PBU** prevents NSIs during use of cannula **4;**
- the position after use **PAU** prevents NSIs after use of cannula **4.**

According to one embodiment, the presence of the lock ring **2** on the sheath of the present invention **1** improves the functional utility and the cost of manufacture. The sheath of the present invention **1** has indeed an optimized size. The sheath of the present invention **1** requires indeed only rotation of the lock ring **2** for reaching the three positions; and in each of said positions, the hub **3** of the sheath **1** is located at the same place.

According to one embodiment, the whole length of the cannula **4** is inside the sheath **1** when the lock ring **2** is in the position before use **PBU** and in the position after use **PAU.** According to one embodiment, the whole length of the cannula **4** is inside the sheath **1** when the lock ring **2** is in the position before use **PBU,** in the position during use **PU** and in the position after use **PAU.** According to one embodiment, when the lock ring **2** is in the position during use **PU,** the cannula **4** may be inside the sheath **1** or may protrudes outside of the sheath 1 if the hub **3,** united with the cannula **4,** is moved in the ejection position **EP** thanks to the ejection means **EM.**

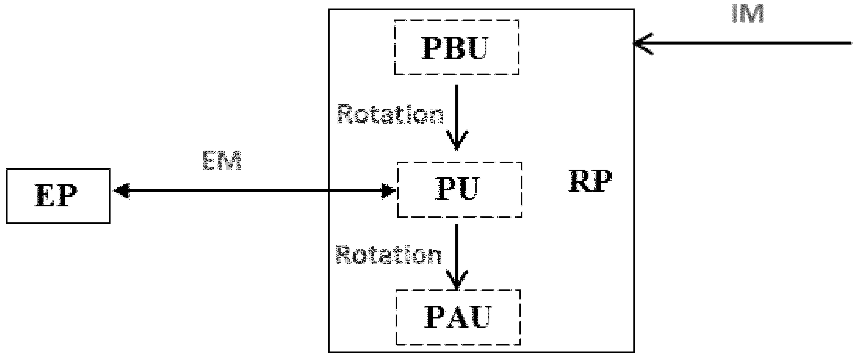

As shown hereabove, a hub **3** may be inserted inside a sheath **1** thanks to an injection means **IM** (described thoroughly hereafter). The hub **3** then reaches a retracted position **RP** in the sheath **1.** In said position **RP,** the whole length of the cannula **4** is inside the sheath **1.** During insertion of the hub **3** inside the sheath **1,** the lock ring **2** is in the position before use **PBU.** In said position **PBU,** the hub **3** cannot reach the distal end **1.8** of the sheath **1** and the cannula **4** is inside the sheath **1** temporarily. Upon rotation of the lock ring **2,** the lock ring **2** rotates from the position before use **PBU** to the position during use **PU.** In said position the cannula **4** is still inside the sheath **1.** However from this position **PU,** the hub **3** may reach an ejection position **EP** thanks to ejection means **EM** (described thoroughly hereafter). In the ejection position **EP,** whole or part of the cannula **4** protrudes outside of the sheath **1.** After use of the cannula **4** in the ejection position **EP,** the cannula may be pulled back inside the sheath and upon rotation of the lock ring **2,** the lock ring **2** rotates from the position during use **PU** to the position after use **PAU.** In said position the cannula **4** is inside the sheath **1** permanently.

In the view of the above, the sheath of the present invention **1** is particularly user-friendly, easy to use and safe for the user, as the only displacements required are rotation of the lock ring **2** with respect to the sheath **1** and translation of the hub **3** with respect to the sheath **1.** According to one embodiment, the use of the sheath **1** does not require rotation and translation of the hub **3** with respect to the sheath **1.**

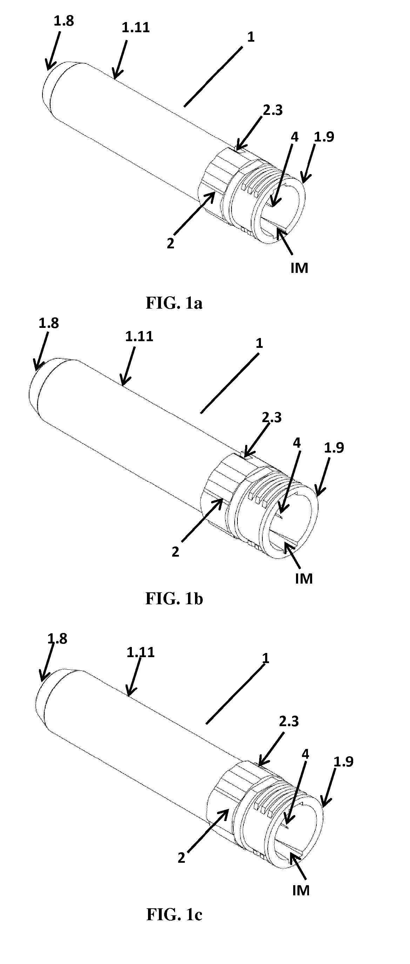

Accordingly figures 1a, 1b, 1c describe a sheath **1** comprising a lock ring **2** rotatably mounted on the outer surface **1.11** of the sheath **1** and a cannula **4.** In figure 1a the lock ring **2** is in the position before use **PBU;** in figure 1b the lock ring **2** is in the position during use **PU;** and in figure 1c the lock ring **2** is in the position after use **PAU.** Figures 1a-c also describes an injection means **IM.**

### Lock ring - recesses

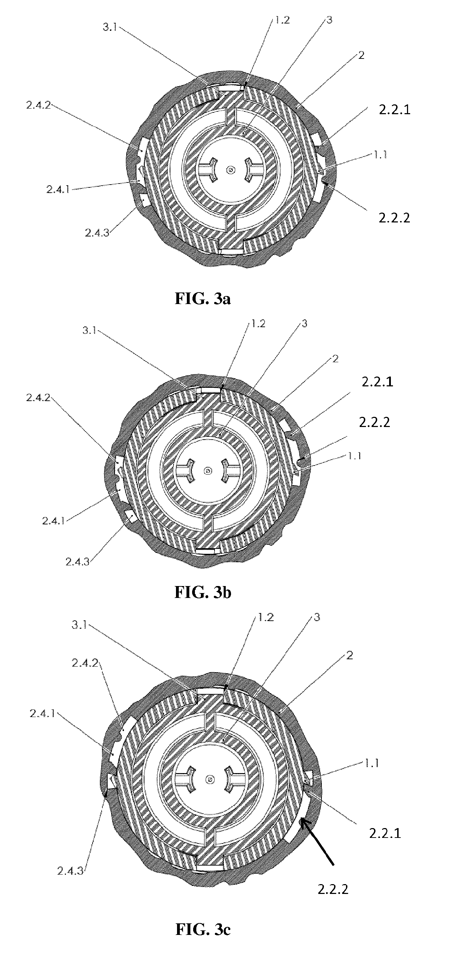

According to one embodiment, the lock ring comprises on the inner surface a first, a second and a third recess (respectively **2.4.1, 2.4.2, 2.4.3).** According to one embodiment, the first, second and third recesses **2.4.1, 2.4.2, 2.4.3** are adjacent and transversally aligned. As detailed in figures 3a-c, the first recess is located between the second and the third recess. Said recesses are designed for cooperating with a protrusion **1.1** on the outer surface **1.10** of the sheath **1.**

According to an embodiment, the protrusion on the outer surface of the sheath **1.1** in cooperation with the three recesses on the inner surface of the lock ring **2.4.1, 2.4.2, 2.4.3** mechanically defines the three positions for the lock ring **2:** a position before use **PBU,** a position during use **PU,** and a position after use **PAU.**

According to one embodiment, the lock ring **2** is designed so that in the position before use **PBU,** the protrusion on the outer surface of the sheath **1.1** is located in a first recess **2.4.1** on the inner surface of the lock ring **2.** According to one embodiment, the lock ring **2** is designed so that in the position during use **PU,** the protrusion on the outer surface of the sheath **1.1** is located in a second recess **2.4.2** on the inner surface of the lock ring **2.** According to one embodiment, the lock ring **2** is designed so that in the position after use **PAU,** the protrusion on the outer surface of the sheath **1.1** is located in a third recess **2.4.3** on the inner surface of the lock ring **2.**

### Sheath

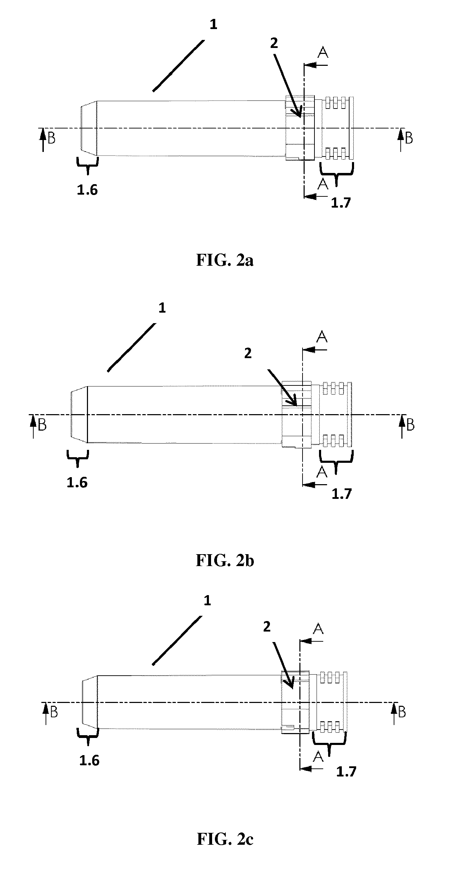

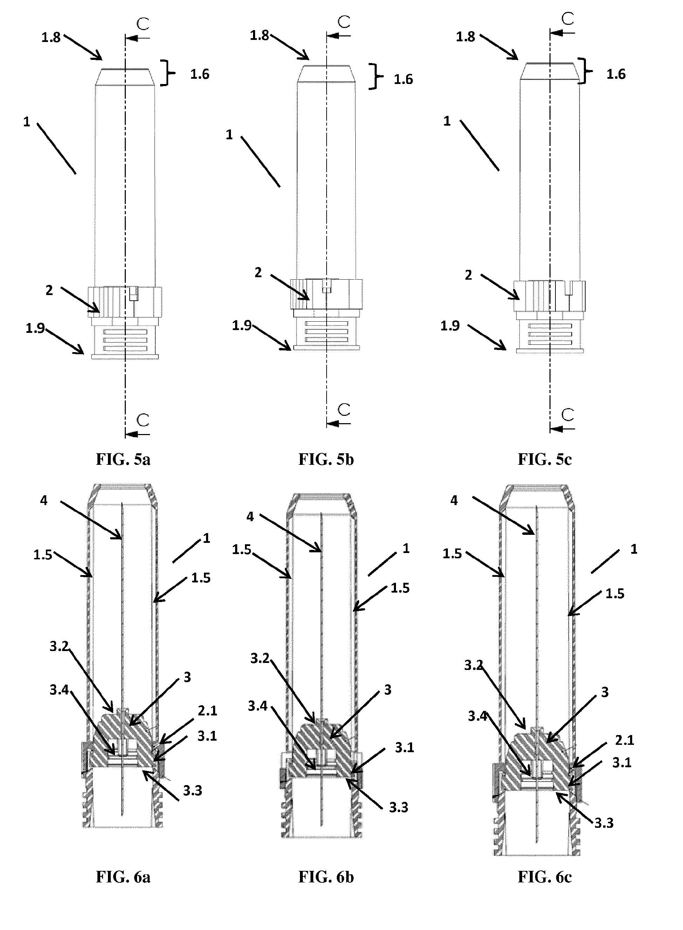

As shown in figures 2a-c and 5a-c, the sheath **1** has an overall shape of a hollow cylinder having a longitudinal axis X; said axis corresponds to the axis B-B in figures 2a-c, and to the axis C-C in figures 5a-c. In an embodiment, the sheath **1** has an internal diameter ranging from 3 to 20 mm, more preferably from 5 to 10mm. In another embodiment, the sheath **1** has an external diameter ranging from 4 to 21 mm, more preferably from 6 to 11mm. In one embodiment, the external and internal diameters of the sheath **1** are preferably substantially constant, at least in a first section of its length. In one embodiment, a substantially constant diameter means that said diameter may vary of maximum 20%, preferably 10%, more preferably 5%, even more preferably 1%, from its nominal value.

In still another embodiment, the sheath **1** has a length ranging from 30 to 250 mm, more preferably from 50 to 200mm, even more preferably from 125 to 175mm.

In one embodiment, the sheath **1** is made of plastic, preferably transparent so that the cannula **4** may be visible through the sheath **1.** In another embodiment, the sheath **1** is opaque. In one embodiment, the thickness of the sheath **1** is preferably substantially constant (for example with a variation of maximum 20%, 10%, 5%, or 1% from its nominal value), but may be narrowed at non-protective locations to save matter, for example at the distal and proximal ends **1.8** and **1.9** of said sheath **1.** In one embodiment, the thickness of the sheath **1** is comprised between 0.2 and 5 mm, preferably between 0.5 and 3 mm, more preferably between 1 and 2 mm, and said thickness may be narrowed of between 20 and 60% at non-protective locations.

### Sheath - ejection means

In an embodiment, the sheath **1** comprises an ejection means **EM,** such as for example a longitudinal slot **1.5** which may cooperate with a protrusion **3.1** of the hub **3** (as shown in figures 6a-c). Said ejection means **EM** allows said hub **3** to switch from a retracted position **RP** to an ejection position **EP,** and conversely. In the retracted position **RP,** the hub **3** is substantially at the proximal end **1.9** of the sheath **1,** so that the whole length of the cannula **4** is inside said sheath **1.** In the ejection position **EP,** the hub **3** is substantially at the distal end **1.8** of the sheath **1,** so that whole or part of the cannula **4** protrudes out of the sheath **1.** Said ejection position **EP** is reachable only from the retracted position **RP** thanks to the ejection means **EM.**

The ejection means **EM** preferably opens to the internal surface **1.10** and not to the external surface **1.11** of the sheath **1.** This feature provides reinforcement for the guiding of protrusion **3.1** with the matter which covers the bottom of the ejection means **EM** and which forms the walls of the ejection means **EM.** However, it is possible to make the ejection means **EM** opens to both internal and external surfaces (respectively **1.10** and **1.11)** of the sheath **1.**

The ejection means **EM** may be any means that one skilled in the art would find suitable such as for example a longitudinal slot **1.5.** The ejection means **EM** comprises preferably a longitudinal slot **1.5** which is only formed on the internal surface **1.10** of said sheath **1.** The ejection means **EM** ends up substantially at the distal end **1.8** of the sheath **1,** approximately at the beginning of the conical distal end, also named beveled portion **1.6** of said sheath **1.** In one embodiment, the protrusion of the hub **3.1** may be stopped by the end of the ejection means **EM** either by the return of the sheath wall inside the sheath **1,** at its distal end **1.8** and/or by anti-slipping means as detailed hereafter.

### Sheath - opening

The sheath **1** also comprises an opening **1.2** for receiving a protrusion **3.1** of the hub **3** (as shown in figures 3a-c). According to a preferred embodiment, the opening **1.2** defines an opening through the internal and external surface of the sheath (respectively **1.10** and **1.11).** According to another embodiment, the thickness of the protrusion **3.1** is equal or slightly smaller than the thickness of the sheath **1.** Thus according to one embodiment, the hub **3** is inserted and slides along the inner surface of the sheath **1.10** by force-fitting until a protrusion of the hub **3.1** reaches the opening **1.2.** According to one embodiment, the hub **3** is in the retracted position with respect to the sheath **1** as long as the hub is located in the opening **1.2.**

According to one embodiment, the opening **1.2** is located in line with the ejection means **EM.** According to one embodiment, the opening **1.2** is located in line with the longitudinal slot **1.5.** According to one embodiment, the ejection means **EM,** preferably the longitudinal slot **1.5,** extends from about the opening **1.2** to about the distal end **1.8** of the sheath **1.**

### Sheath - injection means

According to an embodiment, the hub **3** is inserted through the proximal end **1.9** of the sheath **1** up to the opening **1.2.** According to one embodiment, the hub **3** is inserted and slides along the inner surface of the sheath **1.10** by force-fitting until the hub protrusion **3.1** reaches the opening **1.2.** According to one embodiment, the sheath is provided with an injection means **IM** for assisting in the insertion of the hub **3** inside the sheath **1** (as shown in figures 1a-c). An insertion means **IM** is made by scooping out the interior surface of the sheath **1.10** to obtain a groove opening only on the internal surface of said sheath **1.10.** According to one embodiment, said groove begins about the proximal end **1.9,** preferably at the proximal end **1.9,** and ends at the opening **1.2.** According to one embodiment, said injection means **IM** is in line with the longitudinal slot **1.5** and with the opening **1.2.** According to one embodiment, the injection means is an inclined grove: the depth of said groove varying from a null value about the opening **1.2** to a depth equal or less than the thickness of the hub protrusion **3.1** at the proximal end **1.9.** According to one embodiment, the groove of said injection means **IM,** has a depth increasing gradually as one approaches the proximal end **1.9** of the sheath **1.** According to one embodiment, the width of said groove is equal to about the width of the hub protrusion. According to one embodiment, said injection means **IM** leads to the opening **1.2** which preferably opens both to the internal and external surfaces **1.10** and **1.11** of said sheath **1.** According to one embodiment, said opening **1.2** is located further to the inclined plane of the injection means **IM** toward the distal end **1.8** of said sheath **1.** This feature aims at facilitating the insertion of hub protrusion **3.1** in the sheath **1.**

### Sheath - sliding opening

According to one embodiment, the lock ring **2** is retained with respect to the sheath **1** by means of a lock ring protrusion **2.1.** In one embodiment, said lock ring protrusion **2.1** (i) extends in the direction of the cannula **4,** (ii) is located on the inner surface of the lock ring **2** and/or (iii) cooperates with a sliding opening of the sheath **1.4.**

According to one embodiment, the sliding opening **1.4** is located transversally between the opening **1.2** and the longitudinal slot **1.5.** According to one embodiment, the longitudinal slot extends from the at least one sliding opening **1.4** to about the distal end of the sheath **1.8.**

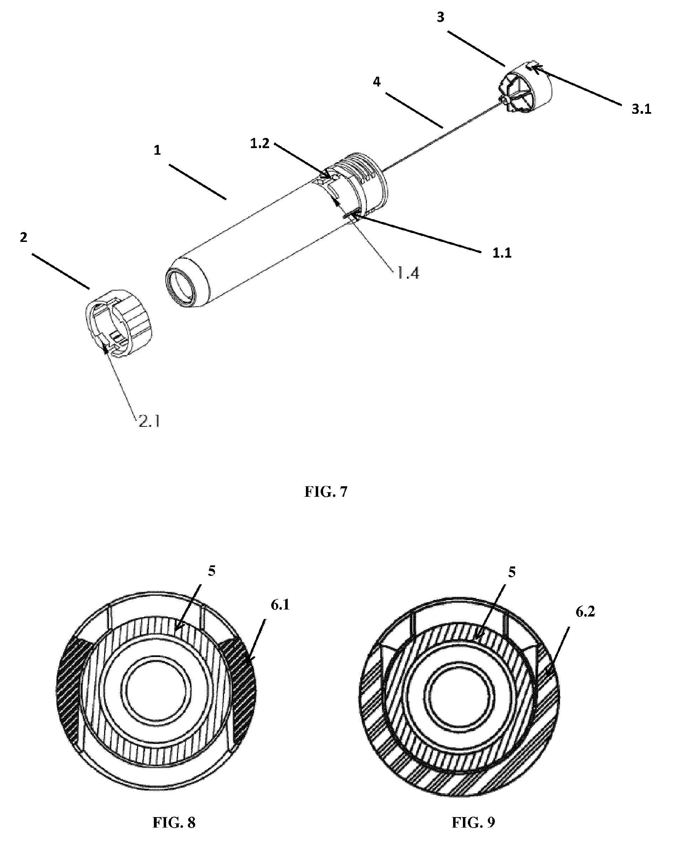

According to one embodiment, the opening **1.2** and the sliding opening **1.4** defines T-shaped slot through the sheath **1;** as shown in figure 7. According to said embodiment, the distal portion of said T-shaped slot corresponds to the sliding opening **1.4** and the proximal portion of said T-shaped slot corresponds to the opening **1.2.**

Figure 7 describes in an exploded view:
- the sheath **1** comprising the opening **1.2,** the sliding opening **1.4** and the sheath protrusion **1.1;**
- the lock ring **2** comprising the lock ring protrusion **2.1** for cooperating with the sliding opening of the sheath **1.4;**
- the hub **3** with a hub protrusion **3.1;** and
- a cannula **4.**

In one embodiment, the lock ring **2** is retained with respect to the sheath **1** when the lock ring protrusion **2.1** is located in the sliding opening **1.4** of the sheath **1.** In an embodiment, the thickness of said lock ring protrusion **2.1** is equal or slightly larger than the thickness of the sliding opening **1.4.** According to a preferred embodiment, the sliding opening **1.4** defines an opening through the internal and external surface of the sheath **1.10** and **1.11** and thus the thickness of the lock ring protrusion **2.1** is equal or slightly larger than the thickness of the sheath **1.** In said embodiment, the lock ring protrusion **2.1** prevents any displacement of the hub **3** towards to distal end **1.8** further to the sliding opening **1.4** when the lock ring protrusion **2.1** is in line with the opening **1.2.**

According to one embodiment, the lock ring **2** is inserted and slides along the outer surface of the sheath **1.10** by force-fitting until it reaches the sliding opening **1.4.**

According to one embodiment, the length of the lock ring protrusion **2.1** in the axial direction is equal or slightly inferior to the length of the sliding opening of the sheath **1.4** in the axial direction. So with the lock ring protrusion **2.1** in the sliding opening of the sheath **1.4,** any axial displacement is prevented.

According to one embodiment, the length of the lock ring protrusion **2.1** in the transversal direction is inferior to the length of the sliding opening of the sheath **1.4** in the transversal direction; thus allowing rotation of the lock ring **2** with respect of the sheath **1.** Said rotation of the lock ring **2** with respect to the sheath **1** corresponds to the rotation of the lock ring protrusion **2.1** inside the sliding opening of the sheath **1.4.** According to a preferred embodiment, the ratio between the length of the sliding opening **1.4** in the transversal direction and the length of the lock ring protrusion **2.1** in the transversal direction is equal to at least 2; more preferably is equal to 2. With the lock ring protrusion **2.1** in the sliding opening of the sheath **1.4,** rotation is allowed within the boundaries of the sliding opening **1.4.** According to one embodiment, the lock ring **2** may rotate with respect to the sheath **1** along the whole sliding opening **1.4.** According to one embodiment, when the lock ring **2** rotates with respect to the sheath **1** between the position before use **PBU,** the position during use **PU** and the position after use **PAU,** the lock ring protrusion **2.1** rotates with respect to the sheath **1** within the sliding opening **1.4.**

In an embodiment, the sheath of the invention **1** is delivered to the user with the lock ring protrusion **2.1** located inside the sliding opening of the sheath **1.4.**

In one embodiment, in order to make easier the localization of the lock ring protrusion **2.1,** the lock ring **2** comprises an opening **2.3** adjacent to the lock ring protrusion **2.1.**

### Effect of the rotation of the lock ring with respect to the sheath

According to one embodiment, in the position before use **PBU,** the protrusion on the inner surface on the lock ring **2.1** temporarily prevents access between the opening **1.2** and the ejection means **EM** (e.g. longitudinal slot **1.5);** as shown in figure 6a. In said embodiment, the hub protrusion **3.1,** located in the opening **1.2,** cannot reach the ejection means **EM** (e.g. longitudinal slot **1.5),** and thus the canula **4** is safely enclosed in the sheath **1.**

In one embodiment, upon rotation of the lock ring **2** in a first direction, the lock ring **2** reaches the position during use **PU.** According to one embodiment, the lock ring **2** is designed so that in the position during use **PU,** the protrusion on the inner surface on the lock ring **2.1** enables access between the opening **1.2** and the ejection means **EM** (e.g. longitudinal slot **1.5);** as shown in figure 6b. In said embodiment, the hub protrusion **3.1,** located in the opening **1.2,** can reach the ejection means **EM** (e.g. longitudinal slot **1.5),** and thus part of the canula **4** can be ejected out of the sheath **1.**

According to one embodiment, in the position during use **PU,** the user may use the ejection means **EM** and slide the sheath **1** with respect to the hub **3** in the axial direction so that whole or part of the cannula protrudes out of the sheath in the ejection position **(EP).** In said embodiment, the hub protrusion **3.1** slides out of the opening **1.2,** through the sliding opening **1.4,** in the longitudinal slot **1.5.** In a preferred embodiment, the sheath 1 does not slide over the lock ring **2** but over the hub **3.**

According to one embodiment, upon rotation of the lock ring **2** in a direction opposite to the first direction, the lock ring reaches the position before use **PU** and then the position after use **PAU.** According to one embodiment, in the position after use **PAU,** the protrusion on the inner surface on the lock ring **2.1** definitely prevents access between the opening **1.2** and the ejection means **EM** (e.g. longitudinal slot **1.5);** as shown in figure 6c. In said embodiment, the hub protrusion **3.1,** located in the opening **1.2,** cannot reach the longitudinal slot **1.5,** and thus the canula **4** is safely enclosed in the sheath **1.**

Figures 6a-c describes a sectional view of the sheath along section plane C-C as defined in figures 5a-c. Figures 6a-c describe a hub **3** comprising a distal end **3.2** and a proximal end **3.3,** a reception means **3.4** and two protrusions **3.1.** Figures 6a-c also describe two longitudinal slots **1.5** on the inner surface of the sheath **1** in line with the hub protrusions **3.1.** In the position before use **PBU** as described in figure 6a, a lock ring protrusion **2.1** prevents access between the opening **1.2,** in which is located the hub protrusion **3.1,** and the longitudinal slot **1.5.** In the position during use **PU** as described in figure 6b, the lock ring protrusion **2.1** has been rotated and does not prevents access between the opening **1.2,** in which is located the hub protrusion **3.1,** and the longitudinal slot **1.5.** In the position after use **PAU** as described in figure 6c, the lock ring protrusion **2.1** has been rotated and prevents access between the opening **1.2,** in which is located the hub protrusion **3.1,** and the longitudinal slot **1.5.**

The disposition of the position before use **PBU,** the position during use **PU,** and the position after use **PAU** of the lock ring **2** also contributes to the healthcare worker's safety. Indeed, according to one embodiment, if the position during use **PU** may be reached from the position before use **PBU** by rotating the lock ring **2** clockwise, respectively anticlockwise, the position after use **PAU** is reached from the position during use **PU** by rotating the lock ring anticlockwise, respectively clockwise. So the healthcare worker cannot reach the position after use **PAU** from the position before use **PBU** inadvertently. Said feature is highlighted by the fact that the first recess on the lock ring **2.4.1** is located between the second recess **2.4.2** and the third recess **2.4.3.** According to an embodiment, the second recess **2.4.2** is not located between the first recess **2.4.1** and the third recess **2.4.3.**

If on the contrary to the present invention, the position during use **PU** could be reached from the position before use **PBU** after a clockwise rotation, respectively an anticlockwise rotation, and the position after use **PAU** could be reached from the position during use **PU** by a clockwise rotation, respectively an anticlockwise rotation; the user could reach the position after use **PAU** from the position before use **PBU** due to a single excessive rotation. Such excessive rotation would prevent the use of the syringe even before any injection.

According to one embodiment, the position after use **PAU** cannot be reached from the position before use **PBU,** through the position during use **PU,** by a continuing rotation. According to one embodiment, the position after use **PAU** can be reached from the position before use **PBU,** through the position during use **PU,** by a continuing rotation

In an embodiment, the sheath **1** of the invention is delivered to the user with the lock ring **2** in the position before use **PBU.**

The rotations of the lock ring **2** with respect to the sheath **1** between the different positions are further illustrated when comparing figures 5a-c. Figures 5a-c describes indeed a sheath **1** according to one embodiment of the present invention. In figure 5a the lock ring **2** is in the position before use **PBU;** in figure 5b the lock ring **2** is in the position during use **PU;** and in figure 5c the lock ring **2** is in the position after use **PAU.** Through these three figures, the rotation of the lock ring is highlighted. Figures 5a-c also point out section plane C-C.

### Lock ring - temporary lock and final lock

As disclosed hereabove, the sheath **1** comprises a protrusion **1.1** on the outer surface for cooperating with the first, second and third recesses (respectively **2.4.1, 2.4.2,** and **2.4.3)** on the inner surface of the lock ring **2**

According to an embodiment, the protrusion on the outer surface of the sheath **1.1** in cooperation with the three recesses on the inner surface of the lock ring **2.4.1, 2.4.2, 2.4.3** mechanically defines the three positions for the lock ring **2:** a position before use **PBU,** a position during use **PU,** and a position after use **PAU.** As detailed in figures 3a-c, the wall between the first recess and the third recess is defined by a lock ring final protrusion **2.2.1;** and the wall between the first recess and the second recess is defined by a lock ring temporary protrusion **2.2.2.**

According to one embodiment, the sheath protrusion **1.1** has a ramp-shaped cross-section. According to another embodiment, the lock ring temporary protrusion **2.2.2** has a curved cross-section, such as a hemisphere. According to another embodiment, the lock ring final protrusion **2.2.1** has a ramp-shaped cross section.

According to an embodiment, the lock ring final protrusion **2.2.1** has a thickness superior to the thickness of the lock ring temporary protrusion **2.2.2.**

According to one embodiment, the sheath protrusion **1.1** on the outer surface of the sheath **1** is designed so that when the lock ring **2** is in the position before use **PBU,** said sheath protrusion **1.1** is located in the first recess **2.4.1** (as shown in figures 3a).

According to one embodiment, when the user rotates the lock ring **2** from the position before use **PBU** to the position during use **PU,** the sheath protrusion **1.1** moves over the lock ring temporary protrusion **2.2.2** and falls into the second recess **2.4.2.**

According to one embodiment, the wall between the first recess **2.4.1** and the second recess **2.4.2** (i.e. the lock ring temporary protrusion **2.2.2)** prevents unintentional displacement of the protrusion **1.1** between said recesses, but enable intentional displacement by application of a torque by the user.

According to one embodiment, when the user rotates the lock ring **2** from the position during use **PU** to the position after use **PAU,** the sheath protrusion **1.1** moves over the lock ring temporary protrusion **2.2.2** and falls into the first recess **2.4.1;** and by further rotation the sheath protrusion **1.1** moves over the lock ring final protrusion **2.2.1** and falls into the third recess **2.4.3.**

According to one embodiment, the sheath protrusion **1.1** and the wall between the first recess **2.4.1** and the third recess **2.4.3** (i.e. the lock ring final protrusion **2.2.1)** is designed so the when the sheath protrusion **1.1** is located in the first recess **2.4.1,** the ramps of the sheath protrusions **1.1** and of the lock ring final protrusion **2.2.1** face each other.

According to one embodiment, the sheath protrusion **1.1** and the wall between the first recess **2.4.1** and the third recess **2.4.3** (i.e. the lock ring final protrusion **2.2.1)** is designed so the when the sheath protrusion **1.1** is located in the third recess **2.4.3,** the radial wall of the sheath protrusions **1.1** and of the lock ring final protrusion **2.2.1** face each other. Therefore, when the lock ring **2** rotates from the position during use **PU** to the position after use **PAU,** the sheath protrusion **1.1,** united with the sheath 1, rotates from the first recess **2.4.1** to the third recess **2.4.3** by moving over the ramp until the sheath protrusion **1.1** falls into the third recess **2.4.3.** Said arrangement is then permanent as the radial surface of the sheath protrusion **1.1** abuts against the radial surface of the lock ring final protrusion **2.2.1**

Figures 3a-c describes a sectional view of the sheath along section plane A-A as defined in figures 2a-c. Figures 3a-c describe a hub **3** comprising a hub protrusion **3.1,** surrounded by a sheath **1** comprising a sheath protrusion **1.1** on the outer surface of the sheath **1.11** and an opening **1.2,** and surrounded a lock ring **2** comprising a lock ring final protrusion **2.2.1** and a lock ring temporary protrusion **2.2.2.** In the position before use **PBU** as described in figure 3a, the sheath protrusion **1.1** is located in the first recess **2.4.1.** In the position during use **PU** as described in figure 3b, the sheath protrusion **1.1** is located in the second recess **2.4.2.** In the position after use **PAU** as described in figure 3c, the sheath protrusion **1.1** is located in the third recess **2.4.3.**

According to one embodiment, the wall between the first recess **2.4.1** and the third recess **2.4.3** (i.e. the lock ring final protrusion **2.2.1)** prevents unintentional and intentional displacement of the protrusion **1.1** between said recesses, once the sheath protrusion is in the third recess **2.4.3.**

In a preferred embodiment, the lock ring **2** of the present invention may take 3 positions with respect to the sheath **1.** In another embodiment, the lock ring **2** of the present invention may take more than 2 positions with respect to the sheath **1.** In an embodiment, the present invention does not include any device featuring two positions only.

In another embodiment, the sheath **1** does not provide a priming position distinct from the ejection position.

In an embodiment, the protrusion of the hub **3.1** is not temporary locked in the ejection position. In another embodiment, the protrusion of the hub **3.1** is temporary locked in the ejection position.

### Use

The passage of the hub **3** between a position outside of the sheath, the retracted position **(RP),** and the ejection position **(EP)** are rendered possible via:
- injection means **IM,** for insertion of protrusion **3.1** of the hub **3** within the sheath **1,** and for guiding said protrusion **3.1** to the opening **1.2,** i.e. to the retracted position **RP;** and
- ejection means **EM,** for guiding said protrusion **3.1** forth from the opening **1.2,** i.e. from the retracted position **RP,** to the ejection position **EP** and back from the ejection position **EP** to the opening **1.2,** i.e. to the retracted position **RP.**

The passage of the lock ring **2** between the position before use **(PBU),** the position during use **(PU)** and the position after use **(PAU)** are rendered possible via:
- rotation in a first direction (clockwise rotation, or respectively anti-clockwise rotation) between the position before use **PBU** to the position during use **PU;**
- rotation in a second direction (anticlockwise rotation, or respectively clockwise rotation) between the position before during use **PU** and the position after use **PAU;**
- said rotation in a second direction also definitely locks the lock ring **2** in the position after use **PAU** in which the protrusion **3.1,** and thus the hub **3,** is blocked in the opening **1.2.**

The injection means **IM** of the sheath **1** provides a one way path for the protrusion **3.1** for inserting the hub from the proximal end of the sheath **1.9** to the opening **1.2;** i.e. to the retracted position **RP.**

When the hub is in the retracted position **RP,** the lock ring **2** may rotate between three positions (position before use **PBU,** position during use **PU** or position after use **PAU).** When the lock ring **2** is in the position during use **PU,** the protrusion of the hub may move from the retracted position **RP** to the ejection position **EP.** In the position after use, the hub is definitely locked in the sheath. The final lock in the position after use **PAU** prevents a cannula **4** equipped with the sheath of the present invention **1** to be reused. According to one embodiment, when the hub **3** is in the retracted position, the whole length of the cannula **4** is inside the sheath **1.**

In one embodiment, the sheath **1** of the present invention does not comprise a transitory position.

Injection means **IM** are preferably one-way transition means, meaning that they allow the transition from a first position to a second position only, but do not allow the reverse transition back from the second position to the first position. These transition means comprise therefore advantageously anti-return means. The achievement of a one-way path ensures an ease of use. In one embodiment, the sheath **1** contains injection means **IM** and ejection means **EM** which provide a precise continuous one-way path for the protrusion of the hub **3.1;** therefore avoiding routing errors and making use easier and safer. In one embodiment, the sheath **1** contains injection means **IM** which provide a precise one-way path for the protrusion **3.1;** therefore avoiding routing errors and making use easier and safer.

In one embodiment, the ejection means **EM** allows reverse transition. In one embodiment, the ejection means **EM** is the only means which allows reverse transition. It is obvious from one skilled in the art that the ejection means **EM** to the ejection position has to allow reverse transition.

A major drawback of using a protective sheath on a cannula is the reduction of manipulation freedom during operation, due to the volume of said sheath.

It has been therefore a goal for the present invention to provide a sheath 1 as short as possible in order to facilitate manipulation during operation, but long enough to ensure a safe manipulation of the device.

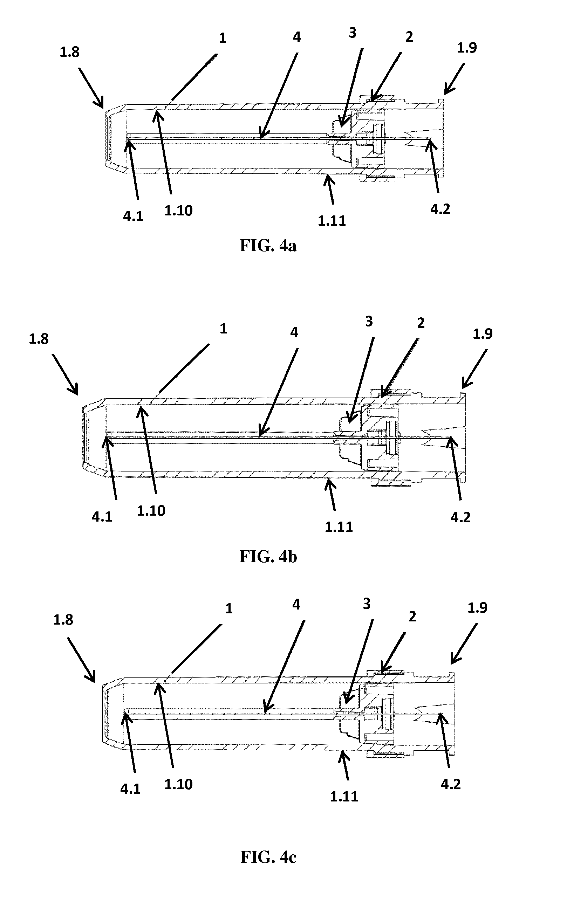

A safe manipulation of a cannula **4** inserted into a sheath **1** is obtained when safety lengths between the sharp edges of the cannula **(4.1** and **4.2)** and the sheath ends **(1.8** and **1.9)** are respected (cf. figures 4a-c). The sheath 1 has therefore to be as long as the cannula length plus the safety lengths. Safety length depends on the diameter of the opening at the sheath ends **(1.8** and **1.9)** from where a cannula end **(4.1** or **4.2)** may be accessed. Typically, a safety length of between 1 and 5 mm, preferably about 3 mm, has to be respected if the diameter of the opening at the sheath ends **(1.8** or **1.9)** is of about 10 mm.

The present invention provides a protective sheath **1** in which the proximal and distal portions of the cannula **4** are protected. For specific applications, the cannula **4** arranged on the hub **3** has to exceed the proximal end of the hub **3.3.** For instance in dentistry, the proximal end of the cannula **4.2,** protruding from the proximal end of the hub **3.3,** perforates the carpule hold in the barrel of a syringe when said syringe is inserted inside the proximal part of the sheath **1.9.** Therefore it is necessary to protect the user from the proximal end of the cannula **3.3.** In the present invention the opening **1.2** which defines the retracted position **RP** is positioned such as the proximal end and the distal end of the cannula **(4.1** and **4.2)** are contained inside the sheath **1.**

In one embodiment of the present invention, the cannula **4** may be totally contained inside the sheath **1.** Especially, in the invention, the protection of the cannula **4** is granted only by the sheath **1** and not by the hub **3,** nor by the lock ring **2.**

In one aspect, the present invention aims at protecting healthcare worker from needlestick injuries.

In an embodiment, the sheath does not to prevent a patient from seeing the needle prior to an injection. In another embodiment, the sheath prevents a patient from seeing the needle prior to an injection.

In one embodiment, regardless of the position of the lock ring **2** (position before use **PBU,** position during use **PU** or position after use **PAU),** the hub **3,** and especially the hub protrusion **3.1** united with the hub **3,** stay in the same position with respect to the sheath. The sheath length can thus be optimized; and the sheath can be the shortest possible while effectively protecting the cannula. Thus, the present invention provides a sheath 1 having an optimized sheath length.

The provision of a short sheath results in a full adaptability of the sheath on the smallest syringes on market when still using a long cannula length.

For the efficacy of the product, the hub **3** has to remain in the sheath **1.** Unfortunately, as a result of the fabrication process, a gap between the sheath **1** and the hub **3** in the locking area cannot be avoided. Thus, a risk exists that protrusion **3.1** on the hub **3** slip under the sheath **1,** which eliminates the safety feature of the device. Therefore, for safety improvement, according to an embodiment, the lock ring protrusion **2.1** has a thickness equal or larger than the thickness of the sheath **1;** thus the lock ring protrusion **2.1** blocks the hub protrusion **3.1** in the position before use and the position after use from inadvertent forth displacement. However to prevent inadvertent backward movement, the sheath **1** according to the invention may further comprise anti-slipping means. In an embodiment, anti-slipping means include means for preventing dismounting of the hub **3** from the sheath **1.** In a preferred embodiment, said anti-slipping means prevents the hub from slipping under the sheath when the hub is moved towards the proximal end of the sheath **1.9.** Although proper use of the device is supposed, the misuse of the device has also to be taken into consideration. In this embodiment, at least one rib may be added, in combination with the hub protrusion **3.1.** This rib is advantageously designed so that once the hub **3** is inserted in the sheath **1** and is passed across said rib, this rib prevents said hub **3** to step back, acting thus as a non-dismountable feature. This rib may be installed near the proximal end of the sheath **1.9,** for example in the first 4 cm, 3cm, 2 cm or 1 cm on the internal surface **1.10** of said sheath **1.** According to one embodiment, anti-slipping means may include inclined plane at the proximal end of protrusion **3.1** and at least one rib toward the distal end **1.8** of said sheath **1** so that protrusion **3.1** are not likely to pass across said rib if a slipping of the hub **3** within the sheath **1** occurs. The angles of the inclined planes are such that when forces are applied in an axial direction, forces perpendicular to the axis force the sheath **1** to get narrower to the hub **3.** The angles range preferably from 30° to 60°, more preferably from 40° to 50°, even more preferably about 45°.Therefore, the slipping of the hub **3** under the sheath **1** is avoided, and the safety is improved.

In another embodiment, anti-slipping means include means for preventing the releasing of the hub out of the sheath when the hub is in the ejection position. In this embodiment, anti-slipping means may include inclined plane at the distal end of protrusion **3.1** and at least one rib, located just next to any hole or groove of the sheath **1** toward the proximal end **1.9** of said sheath **1** so that protrusion **3.1** are not likely to pass across said rib if a slipping of the hub **3** within the sheath **1** occurs. The angles of the inclined planes are such that when forces are applied in an axial direction, forces perpendicular to the axis force the sheath **1** to get narrower to the hub **3.** The angles range preferably from 30° to 60°, more preferably from 40° to 50°, even more preferably about 45°.

Still for a safety improvement, the distal end **1.8** of the sheath **1** is preferably conical or beveled. This shape results on providing a large section in the sheath **1** for a cannula **4** which may be inadvertently bended, and in being tight enough at the distal end **1.8** to avoid the fingers to access said cannula **4.** In one embodiment, the proximal end **1.9** of the sheath **1** is not conical or beveled in order to easy the insertion of the distal end of a syringe. In an embodiment, the protective sheath **1** has a length adapted to protect the user from needlestick.

In one embodiment, the distal end **1.8** of the sheath **1** does not comprise any additional piece, such as a plug, to prevent the hub **3** to step out of the sheath **1.**

In one embodiment, the sheath **1** further comprises gripping means, providing a good grip toward the gloves of healthcare workers. Said gripping means may be for example ribs, preferably covered by a grinding or an erosion grain like VDI 20 to 30. Said gripping means may be located all over the sheath **1** and the lock ring **2,** preferably on the outer surface of the lock ring **2** and on the outer surface of the gripping portion **1.7.**

In an embodiment, every protrusion (on the hub, on the sheath and on the lock ring) and every recess or slot cooperating with said protrusions are arranged in two identical systems diametrically opposite on the hub, on the sheath and on the ring regarding the axis X.

In an embodiment, every protrusion (on the hub, on the sheath and on the lock ring) and every recess or slot cooperating with said protrusions are arranged in 2, 3, 4, 5, or 6 identical systems equally spaced on a diameter of the hub, of the sheath and of the lock ring regarding the axis X.

If more than one system is implemented, all systems are preferably identical.

### Injection system

The present invention further relates to an injection system, comprising a sheath **1** and a hub **3,** inserted within said sheath **1.**

The hub **3** has an overall shape of a cylinder having a longitudinal axis X'. The hub **3** is open at its proximal end **3.3** and is closed at its distal end **3.2** on the cannula **4** or on a cylindrical cavity for the reception of the cannula **4.** As shown in figures 6a-c the hub **3** comprises a reception means **3.4** at its proximal end **3.3,** designed to receive the distal end of a syringe, which is the end farthest to the thumb area of a syringe.

In one embodiment, the reception means **3.4** is a thread allowing a clipping or a screwing of the distal end of a syringe. This thread may be sectioned by two longitudinal slots, for ensuring a clipping by pulling away the two threaded parts forming each a jaw, during the positioning of the hub **3** on the syringe distal end.

In one embodiment, wherein the hub **3** comprises a cannula **4,** said cannula **4** protrudes both distal end **3.2** and the proximal end **3.3** of the hub **3.** When the distal end of a syringe is engaged in the reception means **3.4,** the cannula **4** is in fluid communication with the content of said syringe via its proximal end **4.2.** In other words, the content of the syringe may be flowed through the cannula **4** from the proximal end **4.2.** The content of said syringe may be for example a pharmaceutical composition, which may be contained within a cartridge **5** or the barrel of said syringe, depending on the syringe type. In another embodiment the cannula may comprise two separate parts (i.e. two cannulae) one protruding outside of the distal end of the hub **3.2** and one protruding outside of the proximal end of the hub **3.3;** said two cannulae being fluidly connected inside the hub.

In an embodiment, the hub **3** is used to move between the various position implemented in the sheath **1,** to hold the cannula **4** and, optionally, to fluidly connected the cannulae; however the hub **3** is not design to protect the user from the cannula.

The hub **3** has an external diameter slightly inferior to the internal diameter of the sheath **1.**

In one embodiment, the hub **3** further comprises at least one, or two, three, four, five or six; preferably two protrusions **3.1** diametrically opposite regarding the axis X'. In one embodiment, the hub **3** further comprises at least one protrusion **3.1.** In a preferred embodiment, the hub **3** further comprises at least two protrusions **3.1** diametrically opposite regarding the axis X'. In another embodiment, the hub **3** further comprises at least three protrusions **3.1** regularly spaced on the circumference of the hub **3.** In another embodiment, the hub **3** further comprises at least four protrusions **3.1** regularly spaced on the circumference of the hub **3.** In another embodiment, the hub **3** further comprises at least five protrusions **3.1** regularly spaced on the circumference of the hub **3.** In another embodiment, the hub **3** further comprises at least six protrusions **3.1** regularly spaced on the circumference of the hub **3.** At these protrusions, the diameter of the hub **3** is greater than the internal diameter of the sheath **1,** and preferably substantially identical (permitting a variation of 10%, 5%, or 1% for example), or slightly inferior, to the external diameter of the sheath **1.**

As well known by one skilled in the art, the sheath **1,** the lock ring **2** and the hub **3** are adapted depending on the number of protrusion **3.1.** So in one embodiment wherein the hub comprises 2 protrusions **3.1,** then (1) the sheath **1** comprise, diametrically opposite, two sheath protrusions **1.1,** two openings **1.2,** two sliding openings **1.4,** two longitudinal slots **1.5** and two injection means **IM;** and (2) the lock ring **2** comprises, diametrically opposite, two lock ring final protrusions **2.2.1,** two lock ring temporary protrusion **2.2.2,** two openings **2.3,** and two recesses for the sheath **2.4.1, 2.4.2, 2.4.3** (i.e. 6 recesses). In one embodiment wherein the hub comprises more than two protrusions **3.1,** then (1) the sheath **1** comprise, regularly spaced on the circumference of the sheath **1,** more than two sheath protrusions **1.1,** more than two openings **1.2,** more than two sliding openings **1.4,** more than two longitudinal slots **1.5** and more than two injection means **IM;** and (2) the lock ring **2** comprises, regularly spaced on the circumference of the lock ring **2,** more than two lock ring final protrusions **2.2.1,** more than two lock ring temporary protrusion **2.2.2,** more than two openings **2.3,** and more than two recesses for the sheath **2.4.1, 2.4.2, 2.4.3.**

In an embodiment, the hub **3** is maintained inside the sheath **1** due, in use, to the presence of the protrusion **3.1** inside the thickness of the sheath **1,** preferably inside the opening of the sheath **1.2.**

In an embodiment, the hub **3** does not contain a U-shaped channel or groove around the hub protrusion **3.1.**

The hub **3** is intended to be introduced at the proximal end **1.9** of the sheath **1,** so that the axes X and X' line up. Protrusions **3.1** are thus advantageously adapted to be inserted in female means of the sheath **1.** In one embodiment, protrusions **3.1** of the hub **3** are outwards projections such as bosses. In one embodiment, protrusions **3.1** of the hub **3** are bosses. In an embodiment, the injection system of the invention comprises a sheath **1** and a hub **3** inserted in said sheath **1,** wherein the protrusions **3.1** are in the opening **1.2** of the sheath **1.**

In an embodiment, the injection system of the invention is delivered to the user with the protrusions **3.1** in the opening **1.2.** The injection means **IM** is thus used during the manufacturing and/or assembly of the injection system of the invention.

In an embodiment, the sheath **1** of the present invention is not self-deploying.

### Syringe

This invention also relates to a syringe, preferably to a dental syringe for the injection of a pharmaceutical composition, for instance, of a local anesthetic agent, comprised within a cartridge **5** or the barrel of said syringe; said syringe being equipped with a sheath protecting an injection needle or cannula arranged on a hub, or being equipped with an injection system according to the invention comprising a sheath **1,** a hub **3** and a cannula **4.**

In one embodiment, no other piece than the sheath **1** is intended to protect the cannula **4:** in other words, the sheath **1** is the only safety part for the protection of the cannula **4.**

In one embodiment, no spring is involved in the protection of the cannula **4,** for example in an automatic passage from an extended position to a retracted position, preventing thus a hazardous release of said spring.

The hub **4** is intended to be positioned at the distal end of the syringe, and is aimed at ensuring the entry of the proximal end **4.2** of the cannula **4** within the cartridge **5** or barrel containing the pharmaceutical composition. In one embodiment, the hub **3** is screwed on the distal end of the syringe.

In another embodiment, the hub **3** and the syringe are integrally jointly formed.

In one embodiment, the syringe is a standard syringe.

In another preferred embodiment, the syringe is designed to hold a cartridge **5.**

In the embodiment, wherein the syringe is designed to hold a cartridge **5,** said syringe is equipped with at least one means to hold a cartridge **5.** Preferably, the syringe is equipped in a first section of a first means to hold a cartridge **5,** and is equipped in a second section of a second means to hold said cartridge **5,** said means being complementary to safely hold said cartridge **5.**

In one embodiment, first means to hold the cartridge **5** are self demolding clips **6.1,** presenting a protrusion to hold said cartridge **5,** said protrusion being flexible enough to allow the insertion of said cartridge **5** (as shown in figure 8).

In one embodiment, the second means to hold the cartridge **5** is the bottom of the syringe **6.2,** as described in figure 9.

By these means, the cartridge **5** is totally immobilized in radial direction once inserted into the self demolding clip, avoiding thus to fall out of the syringe.

The cartridge **5** is immobilized in axial direction via a blocking means **6.3.** An axial immobilization is of great importance for the present invention, since active aspiration is therefore allowed by suction of the piston of the syringe. This is particularly useful in dental care, during anesthesia for instance. The dentist loads a cartridge containing the anesthetic agent in a syringe and inserts then the needle tip into the gum. If the needle tip is inserted into a blood vessel, a depression will immediately aspirate blood into the cartridge. The dentist, in order to avoid an injection of anesthetics into a blood vessel, will be averted by the change of the anesthetic color.

Instead of axially fixing the rear of the cartridge **5** as it is the case in common syringes, the cartridge **5** is advantageously maintained at its neck **5.1,** so that the length of the cartridge **5** does no longer play a role in the aspiration process. The syringe of the invention is therefore adaptable on cartridges having different lengths.

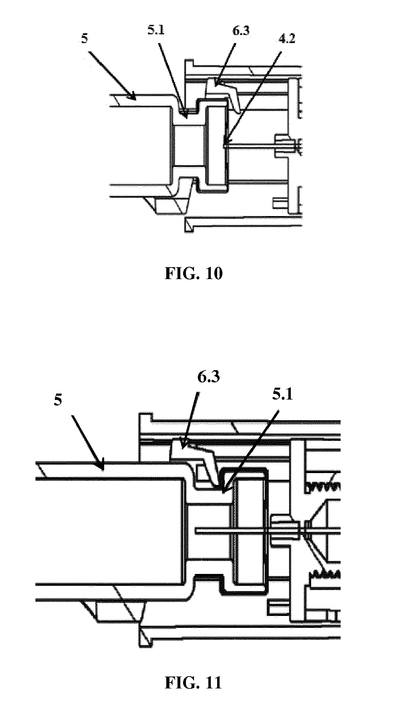

In one embodiment, the blocking means **6.3** is a clip, as shown in figure 10. Preferably, the blocking means **6.3** also immobilizes the cartridge **5** radially.

The blocking means **6.3** is advantageously flexible, allowing the cartridge insertion. When the cartridge **5** is pushed into the end position, the cartridge head will push the blocking means **6.3** up (cf. figure 10). Once the cartridge **5** is in place, the blocking means **6.3** drops back and holds the cartridge **5** at its neck **5.1,** immobilizing thus said cartridge **5** for active aspiration (cf. figure 11).

An advantage of using such a flexible blocking means is the provision of passive aspiration. The blocking means **6.3** may indeed be slightly pushed upward during each pressure on the plunger of the syringe, and will then come back to its initial position creating a depression in the cannula **4.** Preferably, the blocking means **6.3** allows the cartridge **5** to travel between 1 and 5 mm, more preferably between 2 and 4 mm, to assure passive aspiration.

Chirurgical instruments are manipulated with latex gloves. During the operation the gloves get wet and the friction forces of the gloves rubbers are reduced.

When using a plastic part with wet gloves the finger slipping could introduce injuries to the patient. In order to increase the grip, soft overmolding may be installed in portion of the syringe, such as the thumb area, and the grip area. A surface treatment like erosion graining VDI 20 to 30 or surface grindings, nobs or ribs may also be used to increase the surface friction.

Material for these grips may be for example SEBS, TPU, TPE, preferably SEBS (Kraiburg Thermoplast W, Soloplast TH, etc.).

In combination with the gripping means on the gripping portion **1.7** of the sheath **1** and on the lock ring **2,** the syringe of the invention provides a comfortable and safe use.

In one embodiment, the syringe of the invention is a disposable syringe, and allows preferably only a single use.

In this embodiment, the syringe may have the particularity to be conveniently broken after use, in order to separate the sticking part from the non-sticking part. In one embodiment, the sticking part comprises the cannula **4** covered by the sheath **1,** the sheath **1,** the lock ring **2,** the hub **3** and a optionally the distal end of the syringe which is engaged in the reception means **3.4** of the hub **3,** and the non-sticking part comprises substantially the totality of the syringe which may comprise a cartridge **5.**

This has the advantage to reduce the volume of sticking waste having a high risk of contamination, which has to be withdrawn in special containers whose decontamination and destruction are cost intensive. Another advantage is to avoid the congestion of the low volume waste boxes used by medical personal operating in reduced space.

Further to make the end of life of the syringe comfortable, another advantage of such a breakable syringe is the increased difficulty to uncover the cannula **4** after use. The cannula **4** may not be accidentally pushed out of the sheath **1** and become exposed, rendering a needlestick injury even less likely to happen.

A means for an easy and defined breakage is for example a precut, made of a series of perforations, or one or more circumferential grooves, located substantially at the distal end of the syringe, for example in the last 3 cm, 2 cm, 1 cm or 5 mm of said syringe. Preferably, a means for an easy and defined breakage is a circumferential groove.

In the embodiment wherein the syringe is disposable, and wherein the position after use **PAU** is reached via an unscrewing movement or an anti-clockwise rotation, the invention provides a really convenient end of life by an unscrewing movement followed by a break.

In the embodiment wherein the syringe is disposable, and wherein the position after use **PAU** is reached via a screwing movement or a clockwise rotation, the invention provides a really convenient end of life by a screwing movement followed by a break.

In one embodiment, the syringe of the present invention comprises a cartridge **5** which is immobilized in radial direction via demolding clips **6.1** and the back of the syringe **6.2.**

In one embodiment, the syringe of the present invention comprises a plunger equipped with a plunger seal comprising lips, wherein said lips may bend backwards for an easier introduction into the cartridge **5,** said plunger seal being optionally overmolded on the plunger.

In one embodiment, the syringe of the present invention comprises a precut located substantially at the distal end of the syringe, made of a series of perforations or one or more circumferential grooves, preferably one circumferential groove.

This invention further relates to a manufacturing device, preferably a mould or an assembly machine, for manufacturing a sheath **1,** a hub **3,** and/or a syringe according to the present invention.

## Claims

1. A protective sheath (1) for a cannula (4) arranged on a hub which can be fitted onto a syringe **characterized in that**:
- said sheath (1) comprises a lock ring (2) located on the outer surface (1.11) of said sheath (1);
- said lock ring (2) is rotatable with respect to the sheath (1) between three positions:
- a position before use (PBU);
- a position during use (PU); and
- a position after use (PAU).

2. The sheath (1) according to claim **1,** further comprises
- an ejection means (EM) intended to cooperate with a protrusion of the hub (3.1) so that whole or part of the cannula (4) could protrude out of the sheath;
- an opening, in line with the ejection means (EM), for receiving the protrusion of the hub (3.1); and
wherein
- in the position before use (PBU), the lock ring (2) temporarily prevents access between the opening (1.2) and the ejection means (EM);
- in the position during use (PU), the lock ring (2) enables access between the opening (1.2) and the ejection means (EM); and
- in the position after use (PAU), the lock ring (2) definitely prevents access between the opening and the ejection means (EM).

3. The sheath (1) according to claim **2,** wherein the whole length of the cannula (4) is inside said sheath (1) when the protrusion of the hub (3.1) is located in the opening (1.2) of the sheath (1).

4. The sheath (1) according to anyone of claims **1** to **3,** wherein
- the lock ring (2) further comprises a first, a second and a third recess, located on the inner surface of the lock ring (2), and
- the sheath (1) further comprises a protrusion (1.1) on the outer surface (1.11) for cooperating with said recesses;
and wherein,
- in the position before sue (PBU) the protrusion on the outer surface of the sheath is located in a first recess on the inner surface of the lock ring (2);
- in the position during use (PU) the protrusion on the outer surface of the sheath is located in a second recess on the inner surface of the lock ring (2); and
- in the position after use (PAU) the protrusion on the outer surface of the sheath is located in a third recess on the inner surface of the lock ring (2).

5. The sheath (1) according to claim **4,** wherein the sheath protrusion (1.1) has a ramp-shaped cross-section, the wall between the first recess and the third recess has a ramp-shaped cross section, and the wall between the first recess and the second recess has a curved cross-section.

6. The sheath (1) according to claims **4** or **5,** wherein
- the wall between the first recess and the second recess prevents unintentional displacement of the sheath protrusion (1.1) between said recesses, but enable intentional displacement by application of a torque by the user; and
- the wall between the first recess and the third recess prevents unintentional and intentional displacement of the sheath protrusion (1.1) between said recesses, once the sheath protrusion is in the third recess.

7. The sheath (1) according to anyone of claims **2** to **6,** further comprises a sliding opening, located transversally between the opening and the ejection means (EM), for receiving a protrusion located on the inner surface of the lock ring (2.1); and wherein
- when the lock ring (2) is in the position before use (PBU) the protrusion on the inner surface of the lock ring (2.1), located in the sliding opening, prevents access between the opening and the ejection means (EM);
- when the lock ring (2) is rotated in one direction and reaches the position during use (PU) the protrusion on the inner surface of the lock ring (2.1), located in the sliding opening, enables access between the opening (1.2) and the ejection means (EM); and
- when the lock ring (2) is rotate in the opposite direction, the lock ring (2) reaches the position before use (PBU), and by further rotation, the position after use (PAU) and the protrusion on the inner surface of the lock ring (2.1), located in the sliding opening, prevents access between the opening (1.2) and the ejection means (EM).

8. The sheath (1) according to anyone of claims **1** to **7,** further comprising anti-slipping means, including at least one rib for preventing dismounting of the hub (3) from said sheath (1).

9. The sheath (1) according to anyone of claims **1** to **8,** wherein the distal end of said sheath (1.8) is conical or beveled.

10. The sheath according to anyone of claims **1** to **9,** further comprising gripping means which are ribs covered by a grinding or an erosion grain such as VDI 20 to 30, located on the external surface of said sheath (1.11).

11. Injection system comprising:
- the sheath (1) according to anyone of claims **1** to **10,**
- a hub (3), comprising at least one protrusion (3.1) adapted to cooperate with the sheath (1), and
- optionally a cannula (4), protruding at both ends of the hub (3).

12. Syringe comprising a sheath (1) according to anyone of claims **1** to **10** or an injection system according to claim **11.**

13. Syringe according to claim **12** designed to hold a cartridge (5) which is immobilized in axial direction at the neck of said cartridge (5.1), via a blocking means (6.3).

14. Syringe according to anyone of claims **12** or **13,** which is a disposable syringe having a precut located substantially at the distal end of the syringe, made of a series of perforations or one or more circumferential grooves.
